# EUROPÄISCHE PATENTSCHRIFT

(11) **EP 0 519 110 B1**
(45) Veröffentlichungstag und Bekanntmachung des Hinweises auf die Patenterteilung: **17.01.1996**
(21) Anmeldenummer: 91110229.1
(22) Anmeldetag: 20.06.1991
(51) Int. Cl.: G01N 33/48

(54) **Verfahren und Vorrichtung zur schnellen Ermittlung von konzentrationsabhängigen Parametern in einer Messreihe**
Method and apparatus for the rapid determination of concentration dependent parameters in a measurement series
Procédé et dispositif de détermination rapide de paramètres dépendants de la concentration lors d'une série de mesures

(43) Veröffentlichungstag der Anmeldung: 23.12.1992
(73) Patentinhaber: LIST-ELECTRONIC, D-64297 Darmstadt (DE); Krishtal, Prof. Dr., Kiev (SU)
(72) Erfinder: Krishtal, Prof. Dr., Kiev (SU)
(74) Vertreter: Dr. Fuchs, Dr. Luderschmidt Dr. Mehler, Dipl.-Ing Weiss Patentanwälte

(56) Entgegenhaltungen:
- EP-A- 0 128 527
- WO-A-89/10556
- US-A- 4 101 383
- US-A- 4 711 851
- PFLüGERS ARCHIV, Band 391, 1981; O.P. HAMILL et al., Seiten 85-100/

## Beschreibung

Die vorliegende Erfindung befaßt sich mit einem Verfahren und einer Vorrichtung zum schnellen Erfassen konzentrationsabhängiger Parameter in einer Meßreihe mit Lösungen von mit diesen Parametern in Korrelation stehenden chemischen Substanzen, wobei die Meßvorrichtung in die jeweils in Kammern (Schalen) befindlichen Lösungen mit unterschiedlichen Konzentrationen der chemischen Substanz eintauchen und sodann die von der jeweiligen Konzentration der chemischen Substanz abhängigen Parameter bestimmt werden.

Bei der Aufnahme von Meßwerten, bei denen konzentrationsabhängige Effekte miteinander reagierender chemischer Verbindungen oder auf lebende Systeme einwirkender chemischer Verbindungen untersucht werden sollen, steht der Fachmann oft vor dem Problem, daß ein Reaktionsteilnehmer oder Indikator während des Durchlaufs der Meßreihe ständig an Aktivität verliert.

Werden nur Konzentrationsabhängigkeiten mit chemischen Verbindungen gemessen, dann kann das Problem, unter erheblichem Zeitverlust, dadurch umgangen werden, daß jede einzelne Meßcharge kurz vor dem Meßvorgang frisch angesetzt wird oder, wenn es das Abklingverhalten des einen oder anderen Reaktionspartners oder Indikators es zuläßt, daß jeweils nur Teile der Meßreihen neu angesetzt werden; unter Konstanthaltung der äußeren Bedingungen reagieren chemische Verbindungen reproduzierbar gleich miteinander.

Anders verhält es sich bei Einsatz von Bioindikatoren, z.B. in Form von Zellsuspensionen, einzelnen isolierten Zellen oder nur Zellpartikeln. Hierbei ist davon auszugehen, daß die einzelnen Bioindikatoren auf die zu messenden konzentrationsabhängigen chemischen Reize bei definierten äußeren Bedingungen jeweils mit unterschiedlicher Empfindlichkeit ansprechen, das heißt, für die Messungen eines Durchlaufs einer Meßreihe sollte der Bioindikator nicht gewechselt werden.

Beispielhaft dafür steht eine in neuester Zeit entwickelte Methode zur Bestimmung von Dosis-Wirkungs-Beziehungen biologisch wirksamer Substanzen: "Concentration Clamp"- Methode. Darin werden elektrophysiologische Parameter, insbesondere Ionenströme bzw. Ionenleitfähigkeiten, von Membranen isolierter Einzelzellen bzw. Membranflecken in Abhängigkeit von der Konzentration von auf die Zelle oder den Membranfleck einwirkenden biologisch aktiven Substanzen gemessen.

Das Verfahren nutzt die Tatsache aus, daß Zellmembranen im Prinzip elektrische Kondensatoren darstellen, in denen eine Ungleichheit der Ionenverteilung mit Hilfe von ATP-Energie aufrecht erhalten wird. Durch Ionenkanäle (Poren) werden die Ionen mittels für jede Ionensorte spezifischer Carrier (Ionophore) transportiert (biologische Ionophorese). Bei einer Reizung durch chemische Substanzen werden Aktivatoren oder Ionen durch makromolekulare Rezeptoren auf der Membranoberfläche erkannt und zunächst ein Aktivator-Rezeptor Komplex ausgebildet. Dadurch wird ein Aktivierungsmechanismus ausgelöst, in dem die Ionen durch ihnen zugeordnete Ionenkanäle dem Konzentrations- und Ladungsgefälle folgend wieder in die Zelle hinein (Na⁺, Ca⁺) oder hinaus (K⁺)strömen. Infolge davon wird die Membran depolarisiert.

Solche elektrophysiologischen Effekte der Membran als Reaktion elektrischer oder chemischer Reize können quantitativ verfolgt werden, sowohl an isolierten Einzelzellen als auch nur an Membranflecken. Bei der letzteren Methode sitzt auf der Spitze einer Meßpipette (mit einer Öffnung, die in der Regel < = 1µm ist) ein Membranfleck (< =1µ), an dem mit der Meßpipette die potential- und zeitabhängigen Ionenströme gemessen werden (Patch Clamp Methode). Ist der Membranfleck so befestigt, daß die ursprügliche Außenseite der Membran auch nach außen, von der Pipettenspitze wegzeigt, wobei die Innenseite des Membranflecks dann von einer in der Pipette befindlichen, dem intrazellulären Milieu entsprechenden Lösung umspült wird, dann ist es möglich, die Reaktion der Membrankanäle auf Änderungen im extrazellulären Milieu hin über die Bestimmung elektrophysiologischer Parameter messend zu verfolgen (Voltage Clamp-Methode).

Da die biologisch aktiven Substanzen direkt mit den Rezeptoren in Wechselwirkung treten, können somit über Messungen der Membranströme Aussagen über Dissoziations- bzw. Assoziationskonstanten von chemischer Substanz und Rezeptor gewonnen werden, welche auf aktivierende oder desaktivierende Wirkung dieser Substanz schließen lassen. Andererseits können chemische Substanzen direkt mit den Ionophoren (Ionen-Überträgersubstanzen) reagieren und somit den Ionentransport behindern, was auf desensibilierendes oder gar hemmendes Verhalten hinaus läuft. Darüber hinaus lassen sich mit Messungen unterschiedlicher Substanzkonzentrationen Aussagen über die Kinetik der Aktivierung bzw. Desensibilisierung dieser Membranen gewinnen.

Über die Korrelation der dabei gemessenen elektrophysiologischen Parameter in Abhängigkeit von der auf die Zelle bzw. den Membranfleck einwirkenden Konzentration der Substanzen mit bekannten phänotypischen Wirkungen dieser Substanzen lassen sich Voraussagen über die biologische Aktivität von in ihrer Wirkung auf Zellen bisher noch nicht bekannten Substanzen machen, oder es lassen sich Kriterien für die Festlegung von Konzentrationsgrenzen im Hinblick auf Toxizität von noch nicht genauen abzuschätzenden chemischen Verbindungen gewinnen.

Solche Messungen von Konzentrationsreihen chemischer Substanzen mit Bioindikatoren zur Erstellung von Dosis-Wirkungsbeziehungen erfordern aber Voraussetzungen, die nach dem bisherigen Stand der Technik noch nicht alle eingehalten werden konnten:
a) da die aktuelle Vitalität des Bioindikators in Form einer Zelle oder eines Membranflecks stark von der jeweiligen Präparationsmethode, der Geschicklichkeit des Experimentators, der Qualität und dem Alter der jeweiligen Zellsuspension, aus der die Zelle bzw. der Membranlieck heraus präpariert wurde, abhängt, ist aus Gründen der Reproduzierbarkeit eine sinnvolle Aussage und Gegenüberstellung der Meßwerte für die einzelnen Substanzkonzentrationen nur möglich, wenn die gesamte Konzentrationsreihe mit der gleichen Zelle bzw. dem gleichen Membranfleck gemessen wird;
b) da der Bioindikator nur über eine zur Gewinnung sinnvoller Meßdaten begrenzten Lebenszeit verfügt, das heißt, daß seine Vitalität kontinuierlich abnimmt, muß die gesamte Konzentrationsreihe innerhalb dieser Zeitspanne gemessen werden;
c) da sich die optimale Progression der einzelnen Konzentrationen innerhalb einer Konzentrationsreihe, das heißt der Konzentrationsgradient, erst während eines schon stattfindenden Durchlaufs einer Meßreihe ermitteln läßt, sollten einzelne Konzentrationschargen während eines schon stattfindenden Durchlaufs einer Meßreihe austauschbar sein;
d) da die Abnahme in der Vitalität eines Bioindikators vom jeweilig verwendeten Bioindikator und von der jeweiligen Präparation abhängt, sollte der aktuelle Grad der Abnahme der Vitalität des verwendeten Bioindikators während eines Duchlaufs der Konzentrationsreihe ermittelt werden können;
e) da Aussagen über Dosis-Wirkungsbeziehungen umso genauer werden, je mehr Konzentrationen und je öfter die einzelnen Chergen eines jeden Durchlaufs einer Konzentrationsreihe in der zur Verfügung stehenden Zeit gemessen werden können, sollte eine möglichst hohe Durchsatzrate pro Zeiteinheit möglich sein.

Nach dem bisherigen Stand der Technik wurden zu solchen Screening-Versuchen die in Schalen oder Meßröhrchen befindlichen Konzentrationschargen entweder per Hand unter die Vorrichtung befördert, oder es wurden Einheitskassetten mit Reihen von Vertiefungen verwendet in denen die einzelnen verschiedenen Konzentrationen einer Versuchsreihe nacheinander vermessen werden. Die Kassetten mußten zur Wiederverwendung gereinigt werden, wobei Rückstände aus dem Reinigungsprozeß bei der sehr empfindlichen Methode die Meßwerte verfälschen konnten. Darüber hinaus können bei Verwendung dieser Einzelkassetten einzelne Konzentrationschargen während eines Screening-Laufs nicht mehr gegen solche mit geeigneteren Konzentrationen ausgetauscht werden, der Zeitaufwand im Verhältnis zur Lebensfähigkeit des Bioindikators erlaubte nur die Vermessung einer begrenzten Anzahl unterschiedlicher Konzentrationen und die Reproduzierbarkeit der einzelnen Messungen wurde mit zunehmender Zeitdauer, insbesondere gegen Ende des Screening-Tests, praktisch nicht mehr erfüllt.

US-A-4 101 383 und US-A-4 711 851 offenbaren Analyseverfahren, wobei die Proben aufsteuerbaren Tabletts angeordnet sind.

Der Erfindung liegt daher die Aufgabe zugrunde, ein Verfahren und eine Vorrichtung zur Verfügung zu stellen, die es erlauben, einen Screening-Test für konzentrationsabhängige Reaktionen mit einer relativ großen Anzahl gemessener Einzelproben schnell und einfach für solche Reaktionen durchzuführen, bei denen ein Reaktionspartner oder ein Indikator während des Ablaufs der zu testenden Reaktion ständig an Aktivität verliert, die Reaktion damit zeitlich begrenzt ist und nur über einen relativ kurzen Zeitraum messend verfolgt werden kann. Insbesondere soll es damit möglich sein, Reaktionen unter Einsatz von biologischem Material, insbesondere Bioindikatoren in Form von isolierten Zellen oder Zellpartikeln, die im Reaktionsmilieu nur sehr begrenzte "Lebensdauer" aufweisen, unter größtmöglicher Reproduzierbarkeit der Testergebnisse zu messen. Dabei sollen dann die mit der allgemeinen Wirkung der Substanzen auf dem Bioindikator korrelierten Parameter, insbesondere elektrophysiologische Parameter, in Abhängigkeit von der Konzentration einer oder mehrerer chemischer Substanzen gemessen werden können, wobei der Konzentrationsgradient der Testreihe optimal auf die jeweilige Vitalität eines gerade verwendeten Bioindikators eingestellt werden kann.

Diese Aufgabe wird verfahrensmäßig mit den kennzeichnenden Merkmalen des Anspruchs 1 und in Bezug auf die Vorrichtung durch die kennzeichnenden Merkmale des Anspruchs 14 gelöst. Weitere Ausführungsformen sind Gegenstand der Unteransprüche.

Bei der Bestimmung von konzentrationsabhängigen Parametern, insbesondere bei der Erstellung von Dosis-Wirkungsbeziehungen z.B. unter Verwendung von Bioindikatoren mittels der "concentration clamp" Methode stellt sich für den Experimentator zunächst die Frage, wie empfindlich die jeweils als Bioindikator eingesetzte Zellart bzw. der aus der Zellart präparierte Membranfleck auf die jeweils zu messende chemische Substanz anspricht. Die Empfindlichkeit hängt nicht allein von der aktuellen Vitalität des eingesetzten Bioindikators sondern auch von der Affinität der in der Regel in ihrer Wirkung auf den Bioindikator unbekannten chemischen Substanz zu den mit den Ioenkanälen des Bioindikators korrelierten Rezeptoren auf der Membranoberfläche der verwendeten Zellart ab.

Die vorliegende Erfindung bietet nun die Möglichkeit, den optimalen Konzentrationsbereich mit optimalem Gradienten binnen kürzester Zeit zu finden. Die Lösungen mit den einzelnen Konzentrationen werden in Kammern (Schalen) zugegeben, welche dann auf einem Rundtablett in radialsymmetrischer Anordnung mit gleichen Abständen voneinander befestigt werden. Die Befestigung geschieht vorzugsweise mit Klammern, so daß die damit festgehaltenen Kammern jederzeit wieder leicht zu entfernen sind und gegen Kammern mit Lösungen anderer Konzentration und/oder anderer Substanzen ausgetauscht werden können. Das Rundtablett sitzt auf einer senkrechten Antriebswelle auf, welche die Drehbewegung vom computergesteuerten Antriebssystem auf das Rundtablett überträgt. Dieses ist von der Welle leicht abnehmbar, so daß es entweder schnell durch ein Rundtablett mit in Beziehung auf ihre Konzentration anderer Kammeranordnungen bzw. Kammern mit anderen biologisch aktiven Substanzen ersetzt werden kann.

Da die auf dem Rundtablett befindlichen Kammern sowohl einzeln ausgetauscht werden können als auch die Möglichkeit besteht, mehrere unterschiedlich arrangierte Rundtabletts auszutauschen, können binnen kürzester Zeit jeweils beliebige Konzentrationsprofile einer Messung zugeführt werden. Die Einzelmessungen der jeweiligen Konzentrationen nehmen dabei sehr wenig Zeit in Anspruch. Durch das Zusammenfassen von Saugröhrchen und Meßvorrichtung zu einer Meßzelle läßt sich die Geschwindigkeit des einzelnen Meßvorgangs nochmals steigern. Das Einsaugen der jeweils in einer Kammer befindlichen Lösung in das Saugröhrchen bei eingetauchter Meßzelle benötigt nur einige wenige msec (in Abhängigkeit von der Viskosität der Lösung zwischen 2-100 msec), so daß der Bioindikator an seiner aktiven Oberfläche sehr schnell in Kontakt mit der zu messenden Lösung gebracht wird.

Die taktweise horizontale Drehbewegung sowie das Anheben bzw. Absetzen des Rundtabletts oder einzelner Kammern oder das Absenken und Anheben der Meßzellen erfolgen jeweils computergesteuert und sind damit optimal aufeinander abgestimmt.

Ebenso computergesteuert wird die Reihenfolge der Messungen. Werden die Kammern auf dem Rundtablett nacheinander in der Reihenfolge ihrer radialsymmetrischen Anordnung vermessen, dann dreht sich das Rundtablett vor jeder Messung jeweils um den Abstandswinkel zweier benachbarter Kammern. Die vorliegende Erfindung bietet aber auch die Möglichkeit, gemäß einem dem Computer zuvor eingegebenem Programm bzw. durch einen vom Computer aus den zuvor gemessenen aktuellen Daten der Chargen erstellten Reihenfolge, Kammern nacheinander zu vermessen, die in ihrer räumlichen Reihenfolge versetzt voneinander angeordnet sind, wobei sich das Tablett jeweils in der Richtung des kleinsten Abstands der voneinander versetzten Kammern dreht.

Dies bietet insbesondere die Möglichkeit, nach einem zuvor dem Computer eingegebenen zeitlichen oder sequenziellen Abstand, den Inhalt zuvor bereits gemessener Kammern erneut zu vermessen und mit den bereits erfolgten Messungen zu vergleichen. Damit kann das potentielle aktuelle Abklingverhalten der Vitalität des verwendeten Bioindikators beobachtet und dann gegebenenfalls vom Computer bei der Auswertung nachfolgender Messungen berücksichtigt werden.

Insgesamt ist der Zeitaufwand für die vollständige Erfassung einer Meßreihe infolge der computergesteuerten und damit optimal synchronisierten Bewegung des Rundtabletts und/oder der Kammern und/oder der jeweils verwendeten Meßzelle in Verbindung mit dem äußerst schnellen Meßvorgang in der Meßzelle selbst so gering (bei etwa 100 Konzentrationschargen in der Größenordnung von wenigen Minuten), daß die "Lebenszeit" einer isolierten Zelle oder eines isolierten Membranflecks ausreicht, Konzentrationsreihen unterschiedlicher Substanzen mit ein und demselben Bioindikator zu vermessen. Hierdurch werden optimale Aussagen zur vergleichenden Messung biologischer Aktivitäten ermöglicht. Die Einzelkonzentrationen der jeweils zu messenden Substanz werden dabei vor der Messung über einen großen Konzentrationsbereich erstellt, wobei die verwendeten Kammern vorzugsweise aus Einwegmaterial hergestellt sind.

Im folgenden wird der Gegenstand der Erfindung anhand von Zeichnungen näher erläutert:
Es zeigen:

Figur 1 die Anordnung von Meßzelle und Rundtablett, wobei das gesamte Rundtablett in senkrechter Richtung bewegbar ist.

Figur 2 Schnitt durch ein Rundtablett mit Antriebseinheit und Steuereinheit, wobei die Kammern einzeln in senkrechter Richtung bewegbar sind.

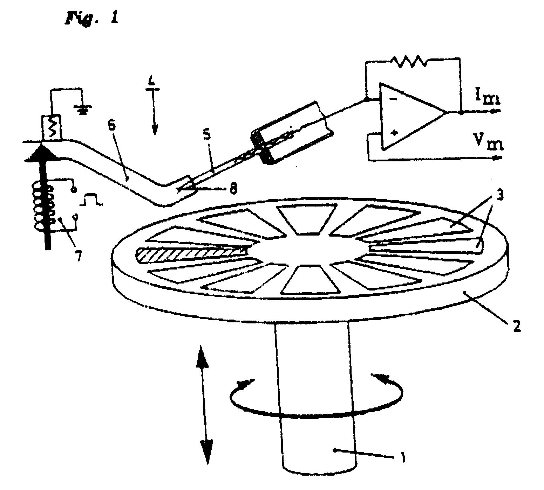

In Figur 1 ist das über die vertikale Welle 1 rotierbare Rundtablett 2 gezeigt, auf dem die einzelnen Kammern 3 radialsymmetrisch angeordnet sind. Die Kammern werden vorzugsweise in ihrer Form angepaßte Vertiefungen eingelassen und durch Klammern gesichert. Die Meßzelle 4, bestehend aus Meßpipette 5 und Saugrohr 6 befindet sich oberhalb des Rundtabletts. Über eine computergesteuerte Prozeßführung wird jeweils die Kammer mit der erwünschten Konzentration an biologisch aktiver Substanz unter die Meßzelle befördert. Sobald sich die Meßzelle über der zu vermessenden Kammer (schraffiert) befindet, wird das Rundtablett um den Betrag nach oben bewegt, bis die Meßzelle 4 in die Kammerlösung eintaucht (nicht eingezeichnet).

Sofort wird die Lösung über das an eine Saugvorrichtung angeschlossenes Saugröhrchen im Zusammenspiel mit dem Ventil 7 angesaugt und der zu bestimmende elektrophysiologische Parameter über die Meßpipette 5 mit anhaftender Zelle 8 oder anhaftendem Membranfleck bestimmt. Der gesamte Meßvorgang dauert nur einige wenige msec. Danach öffnet das Sperrventil 7 wieder und die Lösung wird aus dem Saugröhrchen abgesaugt. Gleichzeitig wird das Rundtablett wieder abgesenkt und gemäß Computerprogramm die nächste Kammer mittels Rotationsbewegung unter die Meßzelle befördert.

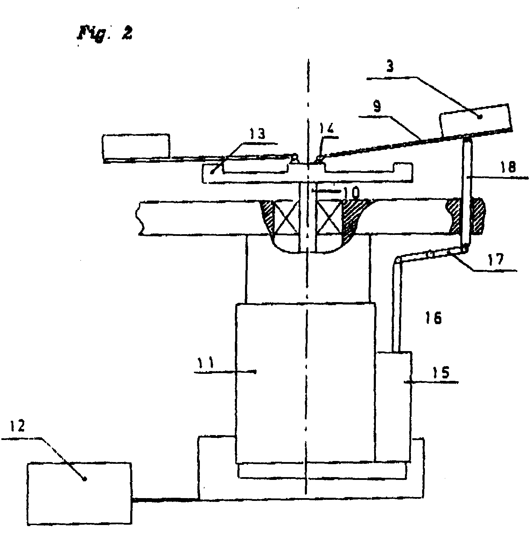

Figur 2 gibt im Schnitt eine Vorrichtung wieder, bei welcher die auf Auslegerarmen 9 befestigten Kammern 3 einzeln senkrecht angehoben und wieder abgesenkt werden können, sobald sie über die Rotationsbewegung des Rundtabletts genau unter der Meßzelle positioniert worden sind.

Das Rundtablett besteht in dieser Ausführungsform aus einer Rotationsscheibe 13, auf welcher die Auslegerarme 9 mit den darauf befindlichen Kammern 3 radialsymmetrisch angeordnet sind. Der Antrieb der Rotationsscheibe 13 erfolgt über die vertikale Welle 10, die ihrerseits von einem Motor, vorzugsweise einem Piezomotor 11 bewegt wird, welcher über eine computerisierte Kontrolleinheit 12 gesteuert wird. Die Verbindung der Auslegerarme 9 mit der Rotationsscheibe 13 erfolgt über Drehgelenke 14 mit horizontalen Drehachsen an den dem Drehpunkt der Rotationsscheibe zugewandten Enden der Auslegerarme 9. Um diese Drehgelenke 14 können die Auslegerarme 9 in vertikaler Bewegungsrichtung geschwenkt werden.

Der Hub der Auslegerarme 9 wird in dieser Ausführungsform über eine Antriebseinheit vermittelt, bestehend aus einer von einem ebenfalls computergesteuerten Antriebsorgan 15 bewegten Hubstange 16, welche die Bewegung über den Hebel 17 auf dem Pusher 18 überträgt, welcher dann schließlich die auf dem Auslegerarm 9 angebrachte Kammer 3 nach oben treibt.

## Patentansprüche

1. Verfahren zur schnellen Ermittlung konzentrationsabhängiger Parameter in einer Meßreihe mit Lösungen von mit diesen Parametern in Korrelation stehenden chemischen Substanzen, wobei die Meßvorrichtung in jeweils in Kammern (Schalen) befindlichen Lösungen mit unterschiedlichen Konzentrationen der chemischen Substanzen eingetaucht wird und sodann die von der jeweiligen Konzentration der chemischen Substanz abhängigen Parameter bestimmt werden,
dadurch gekennzeichnet, daß
a) die einzelnen, jeweils mit einer unterschiedlichen Konzentration an chemischen Substanzen beaufschlagten Kammern auf einem auf eine vertikale Welle aufgesetzten und um diese Welle horizontal rotierbaren und in seiner Bewegung mittels eines computergestützten Systems steuerbaren Rundtablett radialsymmetrisch mit gleichen Abständen voneinander angeordnet werden, wobei die genaue Positionierung der Kammern gemäß den darin enthaltenen Konzentrationen an chemischen Substanzen im Computer eingegeben werden,
b) diejenige Kammer mit der jeweils gemäß Computerprogramm bestimmten Konzentration an chemischen Substanzen zur Bestimmung der mit der Konzentration korrelierten Parameter über eine computergesteuerte Rotationsbewegung des Tabletts direkt unter die oberhalb des Tabletts angebrachte Meßvorrichtung befördert wird,
c) der senkrechte Abstand von Kammer und Meßvorrichtung sodann mittels einer computergesteuerten, senkrecht bewegbaren Hubvorrichtung soweit verringert wird, bis die Meßvorrichtung in die in der Kammer befindliche Lösung eintaucht,
d) nach erfolgter Messung der senkrechte Abstand von Meßvorrichtung und Kammer wieder auf den alten Stand gebracht wird,
e) gegebenenfalls die eine oder mehrere bereits vermessene oder nachfolgende Kammern gegen Kammern höherer oder, je nach Computeranzeige, niedrigerer Konzentration und/oder andere Substanzen ausgetauscht werden, wobei dann das neue Konzentrationsprofil in den Computer eingegeben wird, und
f) zur Messung der gemäß Computerprogramm nächst folgenden Kammer diese wieder über die computergesteuerte Rotationsbewegung des Tabletts um jeweils den Abstandswinkel zweier benachbarter Kammern oder ganzzahliger vielfacher davon unter die Meßvorrichtung befördert wird und die Schritte b) bis e) mit der jeweils im Computer gespeicherten Reihenfolge der Kammern wiederholt wird, bis alle relevanten konzentratsabhängigen Parameter gemäß Computerprogramm erfaßt sind.

2. Verfahren nach Anspruch 1, dadurch gekennzeichnet, daß zum Eintauchen der Meßvorrichtung in die Kammer mit der zu messenden Lösung die Meßvorrichtung während der Bestimmung der Parameter fest an der Analyseapparatur fixiert wird und nachdem die Kammer der jeweils zu bestimmenden Lösung über eine computergesteuerte Rotationsbewegung des Tabletts unter die Meßvorrichtung befördert wurde, das Tablett mit den darauf angeordneten Kammern mittels einer computergesteuerten Hubvorrichtung angehoben und nach erfolgter Bestimmung der jeweiligen Parameter wieder abgesenkt wird.

3. Verfahren nach Anspruch 1, dadurch gekennzeichnet, daß zum Eintauchen der Meßvorrichtung in die Kammer mit der zu messenden Lösung die Meßvorrichtung während der Bestimmung der Parameter fest an der Analyseapparatur fixiert wird und die jeweils mit der gerade zu bestimmenden Lösung beaufschlagte Kammer mittels einer nur der jeweils unterhalb der Meßvorrichtung positionierten Kammer zugeordneten Hubvorrichtung angehoben und nach erfolgter Bestimmung der jeweiligen Parameter wieder abgesenkt wird.

4. Verfahren nach Anspruch 1, dadurch gekennzeichnet, daß zum Eintauchen der Meßvorrichtung in die Kammer mit der zu messenden Lösung nach der genauen computergesteuerten Positionierung dieser Kammer unter die Meßvorrichtung, diese mittels einer am Analysegerät angebrachten Hubvorrichtung zunächst abgesenkt und nach erfolgter Bestimmung der jeweiligen Parameter wieder auf das alte Niveau angehoben wird.

5. Verfahren nach jedem der vorhergehenden Ansprüche, dadurch gekennzeichnet, daß als Kammern zur Aufnahme der Lösungen Einwegartikel verwendet werden.

6. Verfahren nach jedem der vorhergehenden Ansprüche, dadurch gekennzeichnet, daß während eines Durchlaufs der Meßreihe das gerade gemessene Rundtablett durch ein mit noch nicht vermessenen Kammern bestücktes Rundtablett ausgetauscht wird.

7. Verfahren nach jedem der Ansprüche 1-5, dadurch gekennzeichnet, daß während eines Durchlaufs der Meßreihe auf dem Rundtablett befindliche Kammern mit bereits gemessenen Parametern gegen solche Kammern mit noch nicht gemessenen Parametern ausgetauscht werden.

8. Verfahren nach jedem der vorhergehenden Ansprüche, dadurch gekennzeichnet, daß zur Feststellung der Reproduzierbarkeit gemessener Werte die Parameter bereits zuvor gemessener Lösungen nach einer vorbestimmten zeitlichen oder sequenziellen Abfolge erneut gemessen und miteinander im Computer verglichen werden und gegebenenfalls die Werte nachfolgend gemessener Parameter vom Computer entsprechend korrigiert werden.

9. Verfahren nach jedem der vorhergehenden Ansprüche, dadurch gekennzeichnet, daß als Parameter zur Ermittlung von Dosis-Wirkungsbeziehungen biologisch wirksamer Substanzen mit der Wirkung dieser Substanz korrelierte elektrophysiologische Parameter an Zellmembranen gemessen werden.

10. Verfahren nach Anspruch 9, dadurch gekennzeichnet, daß als Meßvorrichtung eine Meßzelle verwendet wird bestehend aus einem an seinem einen Ende an eine Saugvorrichtung angeschlossenen Röhrchen und einer an seiner Pipettenspitze mit einer isolierten Zelle oder einem Membranfleck behafteten mit der Pipettenspitze in das andere Ende des Röhrchens hineinragenden Meßpipette.

11. Verfahren nach Anspruch 10, dadurch gekennzeichnet, daß nach dem Eintauchen der als Meßvorrichtung dienenden Meßzelle in die in der jeweiligen Kammer befindliche Lösung die an das Röhrchen angeschlossene Saugvorrichtung so lange die in der Kammer befindliche Lösung einsaugt, bis die an der Meßpipette haftende Zelle oder der Membranfleck zumindest an der von der Pipettenspitze abgekehrten Seite der Lösung mit der biologisch wirksamen Substanz umspült und dann die entsprechenden elektrophysiologischen Parameter bestimmt werden.

12. Verfahren nach den Ansprüchen 9-11, dadurch gekennzeichnet, daß zur Kontrolle der Vitalität der jeweils als Indikator verwendeten Zelle oder des Membranflecks nach einer vorbestimmten zeitlichen oder sequenziellen Abfolge der Inhalt von diskreten Kammern mit zuvor bereits gemessenen Konzentrationen erneut gemessen wird, die dabei gegebenenfalls auftretenden Differenzen in dem Parameter vom Computer erfaßt werden und die zu beobachtende Abnahme der Vitalität des Bioindikators bei der Auswertung von neu zu bestimmenden nachfolgenden Parametern berücksichtigt werden.

13. Verfahren nach den Ansprüchen 9-13, dadurch gekennzeichnet, daß die Lösung mit der biologisch aktiven Substanz nach dem Eintauchen der Meßzelle in die in der jeweiligen Kammer zu bestimmende Lösung in Abhängigkeit von der jeweiligen Viskosität der Lösung innerhalb von 2-50 msec in das Saugröhrchen gesaugt wird.

14. Vorrichtung zur Durchführung des Verfahrens nach den Ansprüchen 1-13, dadurch gekennzeichnet, daß sie folgende Mittel umfaßt:
(a) ein unterhalb der Meßvorrichtung eines Analysegerätes angeordnetes auf einer vertikalen Welle (1, 10) montiertes und um diese Welle computergesteuert in horizontaler Lage rotierbares Rundtablett (2), auf welchem Kammern (3) für die für einen Meßdurchlauf und einen Teildurchlauf erforderlichen Lösungen mit jeweils unterschiedlichen Konzentrationen an chemischen Substanzen in radialsymmetrischer Ausrichtung mit gleichen Abständen voneinander positionierbar sind, wobei bei jeder Einzelmessung die jeweilige Kammer (3) mit dem zu messenden Inhalt über eine Rotationsbewegung des Rundtabletts (2) genau unter die Meßvorrichtung (4) zu liegen kommt, so daß bei Verringerung des senkrechten Abstandes von Meßvorrichtung und Rundtablett (2) die Meßvorrichtung in die in dieser Kammer befindliche Lösung eintauchen kann,
(b) Mittel zur reversiblen senkrechten Abstandsverringerung von Meßvorrichtung (4) und den auf dem Rundtablett (2) positionierten Kammern (3),
(c) eine computergestützte Steuereinheit (12) zur Steuerung der Rotationsbewegung des Rundtabletts (2) und der darauf abgestimmten reversiblen senkrechten Abstandsverringerung gemäß vorher eingegebenem Computerprogramm.

15. Vorrichtung nach Anspruch 14, dadurch gekennzeichnet, daß die Mittel zur reversiblen senkrechten Abstandverringerung von Meßvorrichtung (4) und Kammern (3) an der senkrechten Welle (1) des Rundtabletts angeordnet sind und die Welle mit dem Rundtablett in senkrechter Richtung nach oben und wieder in den Ausgangszustand nach unten überführbar ist.

16. Vorrichtung nach Anspruch 14, dadurch gekennzeichnet, daß zur Befestigung der Kammern (3) auf dem Rundtablett (2) in radialsymmetrischer Anordnung mit gleichen Abständen voneinander Mittel vorgesehen sind, die ein leichtes Abnehmen der Kammern erlauben.

17. Vorrichtung nach Anspruch 14, dadurch gekennzeichnet, daß das Rundtablett als Rotationsscheibe (13) ausgebildet ist, der ein in Bezug auf deren Drehpunkt radialsymmetrisch ausgerichteter Kranz von Auslegerarmen (9) aufliegt, wobei diese Auslegerarme mit der Rotationsscheibe (13) an dem den Drehpunkt derselben zugewandten Ende jeweils über ein eine horizontale Drehachse aufweisendes Drehgelenk (14) verbunden sind, während das dem Drehpunkt der Rotationsscheibe abgewandte Ende der Auslegerarme jeweils um das Drehgelenk nach oben schwenkbar ist und auf diesem vom Drehpunkt abgewandtem Ende der Auslegerarme (9) jeweils eine Kammer (3) reversibel befestigbar ist und eine Hebevorrichtung für den jeweils unterhalb der Meßvorrichtung befindlichen Auslegerarm (9) samt darauf befindlicher Kammer (3), um diesen Auslegerarm nach oben zu schwenken und nach der Messung wieder abzusenken.

18. Vorrichtung nach Anspruch 17, dadurch gekennzeichnet, daß die Auslegerarme (9) über den Radius der Rotationsscheibe hinausragen und die Hebevorrichtung des jeweils unterhalb der Meßvorrichtung befindlichen Auslegers einen im über den Radius der Rotationsscheibe (13) hinausragenden Bereich des Auslegerarms (9) von unten einwirkenden "Pusher" (18) umfaßt.

19. Vorrichtung nach Anspruch 14, dadurch gekennzeichnet, daß die Mittel zur reversiblen senkrechten Abstandsverringerung von Meßvorrichtung (14) und Kammern (3) an dem Analysegerät angeordnet sind und die Meßvorrichtung (4) senkrecht und reversibel verschiebbar ist.

20. Vorrichtung nach jedem der Ansprüche 14-19, dadurch gekennzeichnet, daß das Rundtablett (2) von seiner senkrechten Antriebswelle (1, 10) abnehmbar ausgebildet ist.

21. Vorrichtung nach jedem der Ansprüche 14-20, dadurch gekennzeichnet, daß die Kammern (3) auf dem Rundtablett (2) bzw, den Auslegern (9) abnehmbar ausgebildet sind.

22. Vorrichtung nach den Ansprüchen 14-21, dadurch gekennzeichnet, daß für den Antrieb zur Rotationsbewegung des Rundtabletts ein Piezomotor vorgesehen ist.

## Claims

1. A process for the quick determination of concentration-dependent parameters in a series of measurements comprising solutions of chemical substances correlated with the said parameters, wherein the measuring device is submerged into solutions of varying concentrations, each solution being contained in a chamber (basin), and subsequently the parameters depending from the respective concentration of a chemical substance are determined,
characterized in that,
a) the individual chambers, each being charged with varying concentrations of chemical substances, are disposed on a round tray at equal distances one from another with rada symmetry, said round tray being mounted on a vertical shaft and being horizontally rotatable round said shaft and, as concerns its movement, being controlled by a computer-aided system, whilst the exact position of said chambers is entered into the computer in accordance with the respective concentrations of chemical substances contained therein,
b) that chamber containing the concentration of chemical substances that is just defined by the computer program is transported directly via a computer-aided gyratory movement beneath the measuring device mounted above the tray in order to determine the parameters correlated with concentration,
c) subsequently, by means of a computer-aided vertically movable lifting device, the vertical distance from the chamber and the measuring device is diminished to such a degree, that the measuring device submerges into the solution contained in the chamber,
d) the measurement being finished, the vertical distance between the measuring device and chamber is returned to the former level,
e) if necessary, one or several already measured or following chambers are exchanged by chambers containing higher or, depending from the indication of the computer, lower concentrations, and/or by other solutions, whilst the new concentration profile is entered into the computer, and
f) in order to measure the next chamber in conformity with a computer program, via the computer-aided gyratory movement of the tray, said chamber is again transported under the measuring device, each chamber by the distance angle of two adjacent chambers or integral multiples thereof, and steps b) to e), each in the order as memorized in the computer are repeated until all the relevant concentration-dependent parameters are recorded in conformity with a computer program.

2. A process according to claim 1, characterized in that, for the immersion of the measuring device into the chamber containing the solution to be measured, said measuring device is firmly attached at the analyzer and after the chamber containing the relevant solution to be determined has been transported under the measuring device by a computer-aided gyratory movement, the tray with the chambers disposed thereon is lifted by means of a computer-aided lifting device, and, the determination of the relevant parameters being finished, is subsequently lowered.

3. A process according to claim 1, characterized in that, for the immersion of the measuring device into the chamber containing the solution to be measured, said measuring device is firmly attached at the analyzer, and each chamber containing just the solution to be determined is lifted by means of a lifting device, which is only allocated to the chamber just disposed below the measuring device and, the determination of the relevant parameters being finished, is subsequently lowered.

4. A process according to claim 1, characterized in that, for the immersion of the measuring device into the chamber containing the solution to be measured, after said chamber has been exactly positioned under the measuring device with the aid of a computer, said chamber is first lowered by means of a lifting device disposed at the analyzer and then lifted up the the former level after determination of the relevant parameter.

5. A process according to any of the previous claims, characterized in that throw-aways are utilized as chambers for the charge with the solutions.

6. A process according to any of the previous claims, characterized in that, during a run of a series of measurements, the round tray just measured is exchanged by a round tray loaded with chambers not yet measured.

7. A process according to any of claims 1 to 5, characterized in that, during a run of a series of measurements, chambers disposed on the round tray and having parameters already measured are exchanged by chambers having parameters not yet measured.

8. A process according to anyone of the previous claims, characterized in that, for the determination of the reproducibility of measured values, the parameters of already previously measured solutions are again measured in accordance with a predetermined temporal or sequential order and are compared one to another in the computer and, if necessary, the values of subsequently measured parameters are accordingly corrected by the computer.

9. A process according to any of the previous claims, characterized in that, as parameters for the determination of dose-effect correlations of bioactive substances, electrophysiological parameters that are correlated with the effect of said substances are measured at the cell membrane.

10. A process according to claim 9, characterized in that a measuring cell is utilized as measuring device comprising a test tube connected at its one end with the suction device and a measuring pipette submerging with its pipette head into the other end of the test tabe and having an isolated cell or membrane patch attached to said pipette head.

11. A process according to claim 10, characterized in that, after immersion of the measuring cell serving as measuring device into the solution contained in the respective chamber, the suction device connected with the test tube imbibes the solution contained in the chamber as long as the cell or the membrane patch attached to the measuring pipette are bathed by the bioactive substance at least at the side opposed to the pipette head, and subsequently the corresponding electrophysiological parameters are determined.

12. A process according to claims 9 to 11, characterized in that, for the examination of the vitality of the cell or membrane patch respectively utilized as indicator, with a predetermined or sequential order, the contents of discrete chambers containing already previously measured concentrations are again measured and the possibly occurring differences with respect to the parameters are determined by the computer and the apparent degrees of the bioindicator vitality is taken into account when former parameters that are newly to be determined are evaluated.

13. A process according to claims 9 - 12, characterized in that, after the immersion of the measuring cell into the solution to be determined in the respective chamber, said solution containing the bioactive substance is sucked within 2 - 50 msec into the suction tube depending from the respective viscosity of the solution.

14. A device to execute the process of claims 1 - 13, characterized in that it comprises the following means:
(a) a round tray (2) disposed beneath the measuring device of an analyser and mounted on a vertical shaft (1,10) and, by the control of a computer, being horizontally rotatable round said shaft, whereas chambers (3) for solutions required for a run or a partial run can be positioned at equal distances one from another in an orientation of radial symmetry, each of the solution containing varying concentrations of chemical substances, wherein at each single measurement, via a gyratory movement of the round tray (2) the respective chamber (3) with the content to be measured will happen to be situated exactly beneath the measuring device (4) so that said measuring device is in the condition to immerse into the solution contained in said chamber when the vertical distance of the measuring device and the round tray (2) is diminished,
(b) means for a reversible diminusion of the vertical distance of the measuring device (4) and the chambers (3) positioned on the round tray (2),
(c) a computer-aided controller (12) to control the gyratory movement of the round tray (2) and the reversible diminution of the vertical distance matched therewith, in accordance with the previously entered computer program.

15. A device according to claim 14, characterized in that the means for the reversible diminusion of the vertical distance of the measuring device (4) and the chambers (3) are disposed at the vertical shaft (1) of the round tray (2) and in that the shaft can be transferred together with the round tray in vertical direction upwards and returned downwards to the initial state.

16. A Device according to claim 14, characterized in that means are provide for the attachment of the chambers (3) and the round tray (2) at equal distances one from another in an arrangement of radial symmetry, said means permitting the chambers to be easily removed.

17. A device according to claim 14, characterized in that the round tray (2) is developped in a form of a rotary disk (13) whereupon there rests a circle of cantilevers (9) organized in radial symmetry with respect to their centre of gyration, each of said cantilevers being connected with the rotary disk (13) at the end facing the centre of gyration thereof by a turning knuckle having a horizontal swivelling axes (14) whilst each end of the cantilevers in opposite direction of the centre of gyration of the rotary disk is pivotable upwards and a chamber (3) is reversibly attachable on said end opposite to the centre of gyration of each cantilever (9), and a lifting device is provided for each cantilever (9) situated beneath the measuring device together with the chamber (3) situated thereupon in order to turn said cantilever upwards and return it after the measurement.

18. A device according to claim 17, characterized in that the cantilevers (9) protect beyond the radius of the rotary disk and that the lifting device of the cantilever respectively sitatuated beneath the measuring device comprises a "pusher" (18) acting from below in the domain of the cantilever (9) that projects beyond the radius of the rotary disk (13).

19. A device according to claim 14, characterized in that the means for the reversible diminution of the vertical distance of the measuring device (14) and the chambers (3) are disposed at the analyser and in that the measuring device (4) can be reversibly displaced in vertical direction.

20. A device according to any of claims 14 - 19, characterized in that the round tray (2) is developped for being removable from its vertical drive shaft (1,10).

21. A device according to any of claims 14 - 20, characterized in that the chambers (3) on the round tray (2) or, respectivelly, the cantilevers (9) are developped for being removable.

22. ADevice according to claims 14 - 21, characterized in that a piezo motor is provided for the drive to cause the motion of the round tray.

## Revendications

1. Procédé de détermination rapide de paramètres, qui dépendent de la concentration, dans une série de mesures avec des solutions de substances chimiques qui sont en corrélation avec ces paramètres, selon lequel on immerge le dispositif de mesure dans des solutions situées respectivement dans des chambres (cuvettes) contenant des concentrations différentes des substances chimiques, puis on détermine les paramètres, qui dépendent de la concentration effective de la substance chimique,
caractérisé en ce que
a) on dispose les différentes chambres, qui sont chargées respectivement par une concentration différente de substances chimiques, avec une symétrie radiale et à des distances réciproques identiques, sur une tablette circulaire, qui est montée sur un arbre vertical et peut tourner horizontalement autour de cet arbre et dont le déplacement peut être commandé au moyen d'un système assisté par ordinateur, le positionnement précis des chambres étant introduit dans l'ordinateur en fonction des concentrations en substances chimiques, que contiennent les chambres,
b) on entraîne la chambre qui possède la concentration en substances chimiques, qui est déterminée respectivement en fonction du programme d'ordinateur, pour la détermination des paramètres corrélés à la concentration, au moyen d'un mouvement de rotation, commandé par ordinateur, de la tablette directement au-dessous du dispositif de mesure monté au-dessus de la tablette, et
c) on réduit ensuite la distance verticale entre la chambre et le dispositif de mesure à l'aide d'un dispositif de levage commandé par ordinateur et déplaçable verticalement, jusqu'à ce que le dispositif de mesure pénètre dans la solution située dans la chambre,
d) une fois effectuée la mesure, on ramène la distance verticale entre le dispositif de mesure et la chambre à son ancienne valeur,
e) éventuellement on remplace une ou plusieurs chambres déjà mesurées ou suivantes par des chambres contenant une concentration supérieure ou, en fonction de l'indication de l'ordinateur, une concentration plus faible et/ou d'autres substances, en introduisant ensuite le nouveau profil de concentration dans l'ordinateur, et
f) pour la mesure de la chambre immédiatement suivante conformément au programme d'ordinateur, on fait avancer cette chambre à nouveau au-dessous du dispositif de mesure, au moyen du mouvement de rotation, commandé par ordinateur, de la tablette sur l'angle respectif séparant deux chambres voisines ou sur un multiple entier de cet angle et on répète les étapes b) à e) avec la succession des chambres, mémorisée respectivement dans l'ordinateur, jusqu'à ce que tous les paramètres importants, qui dépendent de la concentration, soient établis en fonction du programme d'ordinateur.

2. Procédé selon la revendication 1, caractérisé en ce que pour l'immersion du dispositif de mesure dans la chambre contenant la solution à mesurer, on bloque le dispositif de mesure fermement sur l'appareil d'analyse, pendant la détermination des paramètres et, une fois que la chambre contenant la solution de mesure devant être respectivement déterminée a été avancée au-dessous du dispositif de mesure au moyen d'un mouvement de rotation, commandé par ordinateur, de la tablette, on soulève la tablette sur laquelle sont disposées les chambres, à l'aide d'un dispositif de levage commandé par l'ordinateur et on l'abaisse à nouveau après détermination des paramètres respectifs.

3. Procédé selon la revendication 1, caractérisé en ce que pour l'immersion du dispositif de mesure dans la chambre contenant la solution à mesurer, on fixe fermement le dispositif de mesure à l'appareil d'analyse, pendant la détermination des paramètres et on soulève la chambre, chargée respectivement par la solution devant être précisément déterminée, à l'aide d'un dispositif de levage associé uniquement à la chambre positionnée respectivement au-dessous du dispositif de mesure et, une fois effectuée la détermination des paramètres respectifs, on abaisse à nouveau cette chambre.

4. Procédé selon la revendication 1, caractérisé en ce que pour l'immersion du dispositif de mesure dans la chambre contenant la solution à mesurer, après le positionnement précis, commandé par ordinateur, de cette chambre au-dessous du dispositif de mesure, on abaisse tout d'abord ce dispositif à l'aide d'un dispositif de levage monté sur l'appareil d'analyse et, une fois effectuée la détermination des paramètres respectifs, on soulève à nouveau le dispositif de mesure en le ramenant à l'ancien niveau.

5. Procédé selon l'une quelconque des revendications précédentes, caractérisé en ce qu'on utilise des articles à usage unique, comme chambres servant à loger les solutions.

6. Procédé selon l'une quelconque des revendications précédentes, caractérisé en ce que pendant l'exécution d'une série de mesures, on remplace la tablette circulaire précisément mesurée, par une tablette circulaire équipée de chambres non encore mesurées.

7. Procédé selon l'une quelconque des revendications 1-5, caractérisé en ce que pendant l'exécution de la série de mesure, on remplace les chambres situées sur la tablette circulaire et dont les paramètres sont déjà mesurés, par des chambres dont les paramètres ne sont pas encore mesurés.

8. Procédé selon l'une quelconque des revendications précédentes, caractérisé en ce que pour la détermination de la reproductibilité de valeurs mesurées, on mesure à nouveau les paramètres de solution déjà mesurés au préalable, selon un déroulement dans le temps ou un déroulement séquentiel prédéterminé et on les compare entre elles dans l'ordinateur, et les valeurs de paramètres mesurés ensuite sont éventuellement corrigées de façon correspondante par l'ordinateur.

9. Procédé selon l'une quelconque des revendications précédentes, caractérisé en ce qu'on mesure, comme paramètres pour la détermination de relations de dose-action de substances actives du point de vue biologique, des paramètres électrophysiologiques, qui sont corrélés à l'action de ces substances, sur des membranes de cellules.

10. Procédé selon la revendication 9, caractérisé en ce qu'on utilise comme dispositif de mesure une cellule de mesure, qui est constituée par un petit tube raccordé par l'une de ses extrémités à un dispositif d'aspiration, et par une pipette de mesure qui comporte, au niveau de sa pointe, une cellule isolée ou un morceau de membrane et qui pénètre, par sa pointe, dans l'autre extrémité du petit tube.

11. Procédé selon la revendication 10, caractérisé en ce qu'après l'immersion de la cellule de mesure utilisée comme dispositif de mesure, dans la solution située dans la chambre respective, on immerge le dispositif d'aspiration raccordé au petit tube dans une solution située dans la chambre, jusqu'à ce que la cellule, qui adhère à la pipette de mesure, ou le morceau de membrane soit balayé, au moins au niveau sur le côté de la solution, qui est tournée à l'opposé de la pointe de la pipette, par la substance active du point de vue biologique et qu'ensuite les paramètres électrophysiologiques correspondants soient déterminés.

12. Procédé selon l'une quelconque des revendications 9-11, caractérisé en ce que pour le contrôle de la vitalité de la cellule utilisée respectivement comme indicateur ou du morceau de membrane, après un déroulement temporel prédéterminé ou un déroulement séquentiel, on mesure à nouveau le contenu de chambres discrètes contenant des concentrations déjà mesurées auparavant, les différences, qui apparaissent éventuellement, entre les paramètres sont détectées par l'ordinateur et la baisse devant être observée de la vitalité de l'indicateur biologique est prise en compte lors de l'évaluation de paramètres suivants devant être à nouveau déterminés.

13. Procédé selon l'une quelconque des revendications 9-13, caractérisé en ce qu'on aspire dans le petit tube d'aspiration, en l'espace de 2-50 ms, la solution contenant la substance active du point de vue biologique, après immersion de la cellule de mesure dans la solution devant être déterminée dans la chambre respective, en fonction de la viscosité respective de la solution.

14. Dispositif selon l'une quelconque des revendications 1-13, caractérisé en ce qu'il comprend les moyens suivants :
a) une tablette circulaire (2), qui est disposée au-dessous d'un dispositif de mesure d'un appareil d'analyse, est montée sur un arbre vertical (1,10) et peut tourner en position horizontale autour de cet arbre, d'une manière commandée par ordinateur, et sur laquelle peuvent être positionnés d'une manière alignée avec une symétrie axiale et à des distances réciproques identiques, des chambres (3) pour les solutions, qui sont nécessaires pour un cycle de mesure et un cycle partiel, et contenant des concentrations respectivement différentes de substances chimiques, auquel cas lors de chaque mesure individuelle, la chambre respective (3) contenant le contenu à mesurer venant s'appliquer exactement au-dessous du dispositif de mesure (4), sous l'effet d'un mouvement de rotation de la tablette circulaire (2), de sorte que, lors d'une réduction de la distance verticale entre le dispositif de mesure et la table circulaire (2), le dispositif de mesure peut pénétrer dans la solution située dans cette chambre,
b) des moyens pouir réduire verticalement, de façon réversible, la distance entre le dispositif de mesure (4) et les chambres (3) positionnées sur la tablette circulaire (2),
c) une unité de commande (12) commandée par ordinateur et servant à commander le mouvement de rotation de la tablette circulaire (2) et de la réduction réversible de distance verticale, accordée sur ce déplacement, conformément à un programme d'ordinateur introduit au préalable.

15. Dispositif selon la revendication 14, caractérisé en ce que les moyens de réduction verticale réversible de la distance entre le dispositif de mesure (4) et les chambres (3) sont disposés sur l'arbre vertical (1) de la tablette circulaire et que l'arbre équipé de la tablette circulaire peut être déplacé verticalement selon un mouvement ascendant et être ramené par abaissement dans l'état initial.

16. Dispositif selon la revendication 14, caractérisé en ce que pour la détermination des chambres (3) sur la tablette circulaire (2), des moyens permettant un retrait facile des chambres, sont disposés selon une disposition à symétrie radiale en étant séparés par des distances réciproques identiques.

17. Dispositif selon la revendication 14, caractérisé en ce que la tablette circulaire est agencée sous la forme d'un disque rotatif (13), sur lequel est disposée une couronne de bras en console (9), qui est disposée avec une symétrie radiale par rapport au centre de rotation, ces bras en console étant reliés, au niveau de leur extrémité tournée vers le centre de rotation, au disque rotatif (13), respectivement par l'intermédiaire d'une articulation pivotante (14) possédant un axe horizontal de rotation, tandis que l'extrémité des bras en console, qui est tournée à l'opposé du centre de rotation du disque rotatif, peut basculer vers le haut respectivement autour de l'articulation pivotante et que respectivement une chambre (3) peut être fixée de façon réversible sur cette extrémité des bras en console (9), tournée à l'opposé du centre de rotation, et qu'il est prévu un dispositif de levage pour le bras en console (9) qui est situé respectivement au-dessous du dispositif de mesure, pour faire basculer vers le haut ce bras en console et l'abaisser à nouveau après la mesure.

18. Dispositif selon la revendication 17, caractérisé en ce que les bras en console (9) font saillie au-delà du rayon du disque rotatif et que le dispositif de levage du bras en console situé respectivement au-dessous du dispositif de mesure comprend un poussoir (18) qui agit à partir du bas sur la partie du bras en console (9), qui fait saillie au-delà du rayon du disque rotatif (13).

19. Dispositif selon la revendication 14, caractérisé en ce que les moyens pour réduire de façon réversible la distance verticale entre le dispositif de mesure (14) et les chambres (3) sont montés sur un appareil d'analyse et que le dispositif de mesure (4) est déplaçable verticalement et de façon réversible.

20. Dispositif selon l'une quelconque des revendications 14-19, caractérisé en ce que la tablette circulaire (2) est agencée de manière à pouvoir être retirée de son arbre vertical d'entraînement (9,10).

21. Dispositif selon l'une quelconque des revendications 14-20, caractérisé en ce que les chambres (3) situées sur la tablette circulaire (2) ou sur les bras en console (9) sont agencées de manière à pouvoir être retirées.

22. Dispositif selon les revendications 14-21, caractérisé en ce que pour l'entraînement en rotation de la table circulaire, il est prévu un moteur piézoélectrique.
